# EUROPÄISCHE PATENTANMELDUNG

(11) **EP 0 832 985 A2**
(43) Veröffentlichungstag der Anmeldung: **01.04.1998**
(21) Anmeldenummer: 97116616.0
(22) Anmeldetag: 24.09.1997
(51) Int. Cl.: C12Q 1/28, C12Q 1/42

(54) **Enzymatisches Verfahren zum Nachweis der Erhitzungsbehandlung von Milch und Milchprodukten**

(30) Priorität: 26.09.1996 DE 19639538
(71) Anmelder: BOEHRINGER MANNHEIM GMBH, 68298 Mannheim (DE)
(72) Erfinder: Fischer, Jutta, 82362 Weilheim (DE); Von Der Eltz, Herbert, Dr., 82362 Weilheim (DE)

(57) **Zusammenfassung**

Die Erfindung betrifft ein Verfahren zum Nachweis der korrekt durchgeführten Erhitzungsbehandlung von Milch. Insbesondere wird hierzu der Erhitzungsgrad von Milch bzw. Milchprodukten mit Hilfe eines enzymatischen Verfahrens bestimmt. Das Verfahren, das mindestens ein milchspezifisches Enzym zur Bestimmung des Erhitzungsgrades von Milch und Milchprodukten nutzt, wird vorteilhafterweise auf einem festen Träger durchgeführt, wobei ein präzipitierendes Farbstoff bildendes Enzymsubstrat verwendet wird.

## Beschreibung

Die Erfindung betrifft ein Verfahren zum Nachweis der korrekt durchgeführten Erhitzungsbehandlung von Milch und Milchprodukten. Insbesondere wird hierzu der Erhitzungsgrad von Milch bzw. Milchprodukten mit Hilfe eines enzymatischen Verfahrens bestimmt. Das Verfahren, das mindestens ein milchspezifisches Enzym zur Bestimmung des Erhitzungsgrades von Milch und Milchprodukten nutzt, wird vorteilhafterweise auf einem festen Träger durchgeführt.

Ein großer Teil der Milch wird vor der Weiterverarbeitung bzw. Abgabe an den Verbraucher einer Wärmebehandlung unterzogen, um die für den Menschen pathogenen Keime sowie einen Teil der Verderbniserreger abzutöten. Die thermische Behandlung hat daneben auch Veränderungen weiterer Milchbestandteile wie z.B. die Inaktivierung von Enzymen zur Folge. Die Inaktivierung solcher milcheigenen Enzyme dient daher als Nachweis für die ordnungsgemäße Durchführung von Wärmebehandlungsverfahren. Zu den milcheigenen Enzymen, deren Inaktivierung als ein solcher Nachweis gilt, gehören die milcheigene Peroxidase, die sogenannte Lactoperoxidase und die Alkalische Phosphatase. Anerkannte Wärmebehandlungsverfahren sind die Pasteurisierung, die Ultrahocherhitzung und die Sterilisierung. Bei der Pasteurisierung unterscheidet man die Dauererhitzung, die Kurzzeiterhitzung sowie die Hocherhitzung. So ist eine negative Lactoperoxidaseprobe als Nachweis für die ordnungsgemäße Pasteurisierung der Milch durch Hocherhitzung vorgeschrieben. Die alkalische Phosphatase wird zum Nachweis der korrekten Dauererhitzung oder Kurzzeiterhitzung der Milch verwendet; hiernach ist die Milch phosphatasenegativ und peroxidasepositiv. Dahingegen sind nach der Pasteurisierung durch Hocherhitzung, Ultrabocherhitzung oder Sterilisation beide Enzyme inaktiv.

Zum Nachweis der Aktivität milcheigener Enzyme werden üblicherweise Substrate eingesetzt, die nach enzymatisch katalysierter Reaktion (z.B. Redoxreaktionen, Hydrolysen) eine detektierbare Färbung der Reaktionslösung bewirken. Die verschiedenen im Stand der Technik gebräuchlichen Methoden unterscheiden sich hinsichtlich des eingesetzten Substrates sowie hinsichtlich der Probenvorbereitung und der verwandten Detektionstechniken. Diesen Methoden ist gemeinsam, daß sie in Lösung durchgeführt werden und daß sowohl die Probenvorbereitung, als auch die Detektion der Färbung zeitaufwendige Prozeduren und apparativen Aufwand erfordern.

Zur Lactoperoxidaseaktivitäts-Bestimmung wurden bisher zahlreiche Substrate verwendet, wie beispielsweise P-Phenylendiamin (insbesondere die sogenannte Storch'sche Reaktion (Storch, V. (1899) Zeitschrift für Untersuchung der Nahrungs- und Genußmittel **2**, 239-242), Guajakonsäure, Guajakol, Benzidin.

Eine ausreichende Qualität und damit eine amtliche Zulassung als Nachweisreagenz für die ordnungsgemäße Pasteurisierung der Milch durch Hocherhitzung besitzen lediglich das Reagenz Guajak-Reagenz-Neu (Guajakonsäure; Schwarz, G und Sydow, G. (1947) Milchwissenschaft **2**, 424-433), das Hocherhitzungsreagenz N3 (Guajakol; Schwarz, G und Sydow, G. (1947) Milchwissenschaft **2**, 424-433), das Traventol-Reagenz (Guajakol; Deutsches Patent 814670) sowie ein Reagenz auf Basis von 1,4-Phenylendiamin (Storch'sche Reaktion, Storch, V. (1899) Zeitschrift für Untersuchung der Nahrungs- und Genußmittel **2**, 239-242).

Diese Reagenzien besitzen eine Empfindlichkeit, mit der sich eine nach gesetzlichen Vorschriften durchgeführte Hocherhitzung nachweisen läßt. Die Verwendung anderer Reagenzien kann insbesondere aufgrund von Nebenaktivtäten zu falschen Urteilen führen (Schulz, M.E. (1965) Das große Molkereilexikon, Hrsg. Schulz, M.E. und Voss, E., Volkswirtschaftlicher Verlag GmbH, Deutsche Molkerei-Zeitung, Kempten/Allgäu).

Die Detektion des Farbumschlages bzw. die Auswertung der Farbintensität wird bei den meisten im Stand der Technik beschriebenen Verfahren mit Hilfe photometrischer Methoden durchgeführt, was einen nicht zu vernachlässigenden apparativen und zeitlichen Aufwand bedeutet (z.B. EP 0 401813). Üblich ist auch der Vergleich der Farbintensität der Lösung mit einer Reihe von Peroxidase-Standardlösungen. Peroxidase-Standardlösungen enthalten in der Regel Peroxidase aus Meerrettich. Die Meerrettich-Peroxidase ist jedoch hinsichtlich Reaktivität und Aktivität unterschiedlich zu Lactoperoxidase. Damit ist die Vergleichbarkeit von Meerrettich-POD mit Lactoperoxidase nicht gegeben. Der Nachweis der Aktivität der Lactoperoxidase mit Hilfe der Storch'schen Reaktion (amtl. Methode § 35 LMBG) hat zu den bereits genannten, noch den weiteren Nachteil, daß der Farbumschlag nach exakt 30 Sekunden erfolgen muß. Ein früher oder später auftretender Farbumschlag gilt als unspezifische Reaktion.

Zur Bestimmung der Aktivität der milcheigenen Alkalischen Phosphatase werden heute insbesondere die Substrate p-Nitrophenylphosphat und das Fluorophos^{(R)} - Substrat verwendet.

P-Nitrophenylphosphat (Beutler, H.O. (1988) amtl. Methode § 35 LMBG) ist als Nachweisreagenz für die ordnungsgemäße Dauer- und Kurzzeiterhitzung der Milch amtlich zugelassen. Auch bei dieser Methode erfolgt die Auswertung mit Hilfe photometrischer Methoden und erfordert eine aufwendige Probenvorbereitung zur Erreichung eines klaren Filtrates (z.B. mit Detergens Genapol X) und zudem apparativen Aufwand. Die Aktivität der alkalischen Phosphatase wird bei Verwendung des Fluorophos^{(R)} -Substrates mit Hilfe eines kontinuierlichen kinetischen Assays fluorimetrisch gemessen. Der apparative und experimentelle Aufwand ist auch bei dieser Methode groß. In DE 1 285 217 wird die Verwendung von p-Nitrophenylphosphat oder dessen Alkalisalzen zur Vorimprägnierung eines Reagenzpapieres beschrieben, das dann zum Nachweis der alkalischen Phosphatase verwendet wird. Das Verfahren zur Imprägnierung sowie die Aufbewahrung des imprägnierten Papieres ist jedoch sehr aufwendig. Zudem hat das in DE 1 285 217 beschriebene Verfahren den Nachteil, daß in diesem Testsystem kein quantitativer Nachweis der Enzymaktivität möglich ist.

Der vorliegenden Erfindung lag somit die Aufgabe zugrunde, ein enzymatisches Nachweisverfahren zur Ermittlung der korrekt durchgeführten Erhitzungsbehandlung für Milch und Milchprodukten bereitzustellen, wobei als Qualitätsmarker die Aktivität von mindestens einem milchspezifischen Enzym von Bedeutung ist, und wodurch die Nachteile bekannter Methoden zum Erhitzungsnachweis überwunden werden.

Diese Aufgabe wird erfindungsgemäß durch ein enzymatisches Verfahren gelöst, welches im wesentlichen dadurch gekennzeichnet ist, daß eine entsprechende Probe in einer gepufferten Lösung mit einem oder mehreren milchspezifischen - präzipitierenden Farbstoff bildenden - Enzymsubstraten auf einem festen Träger vereinigt wird und als Aktivitätsdetektion die Farbintensität der Enzymspots mit denen einer Reihe von milcheigenen Enzym-Standards verglichen wird. Die zum Nachweis herangezogenen milcheigenen Enzyme sind die Lactoperoxidase und / oder die Alkalische Phosphatase.

Als Vorteile des erfindungsgemäßen Verfahrens erwiesen sich im wesentlichen die folgenden:
- Geräte- und Laborunabhängigkeit, d.h. geringer apparativer und experimenteller Aufwand sowohl bei der Probenvorbereitung als auch bei der Detektion
- Erhöhung der Aussagekraft gegenüber herkömmlichen Tests, indem milcheigene Enzym-Standardlösungen verwendet werden
- ausreichende Empfindlichkeit des Nachweises
- Linearität im Bereich der Empfindlichkeit
- hohe Spezifität des Nachweises, so daß Milchinhaltsstoffe den Test weder stören noch inhibieren
- die Aktivität der milcheigenen Enzyme kann quantitativ bestimmt werden.

Eine ausreichende Empfindlichkeit des erfindungsgemäßen Lactoperoxidase-Nachweises liegt in dem Bereich von ca. 4 U/ml bis 400 U/ml, am besten ist der Bereich von 2 U/ml bis 150 U/ml. Der Empfindlichkeitsbereich des Nachweises der Alkalischen Phosphatase liegt zwischen ca. 3.5 mU/ml und 2 U/ml.

Substrate der Lactoperoxidase, die erfindungsgemäß geeignet sind, sind Naphtole, besonders bevorzugt ist 4-(1,4,7,10-Tetraoxadecyl)-1-naphtol. Als Substrate für die Alkalische Phosphatase haben sich Indole, besonders 5-Brom-4-chlor-3-indolyl-phosphat (BCIP) zusammen mit Nitroblautetrazoliniumchlorid (NBT) als Farbverstärker, als geeignet erwiesen.

4-(1,4,7,10-Tetraoxadecyl)-1-naphtol findet als Meerrettich-Peroxidasesubstrat bereits Anwendung bei Enzym-Immunoassays, Protein-Blots, Antikörper-beschichteten 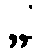 Beads" und DNA- oder RNA-Hybridisierungsblots. NBT/BCIP findet als Substrat der Alkalischen Phosphatase bereits Anwendung in Form von Ready-to-use-Tabletten bei Blotting-Techniken (Southern, Northern, Western), in der Immunohistochemie/Immuncytochemie. Entsprechende Produkte sind bereits auf dem Markt. Die Verwendung der genannten Substrate sind somit aus klinischen Anwendungen bekannt; allerdings lediglich in Form von Immunoassays bzw. fluorimetrischen Testführungen (EP 0 350 808, EP 0 269 979, EP 0 228 663).

Der Aktivitätsnachweis der Lactoperoxidase wird in einem pH-Bereich von 5,5 - 7,0 durchgeführt, bevorzugt ist der Bereich von pH 6,5 - 7,0, als ganz besonders geeignet zeigte sich ein pH-Wert von ca. 6,5. Der pH-Wert der Probelösungen und der Standardlösungen wird durch einen Puffer gewährleistet. Mögliche Puffer sind z.B. K-Phosphat-Puffer oder Na-Phosphat-Puffer oder Mischungen aus beiden. Als besonders geeignet erwies sich ein Na-Phosphat-Puffer. Eine Konzentration der Pufferlösung im Bereich von ca. 40 mM bis 65 mM haben sich erfindungsgemäß als geeignet erwiesen, bevorzugt ist eine Konzentration von ca. 50 mM.

Die Anwesenheit von Wasserstoffperoxid in einer Konzentration von ungefähr 20 - 30 % hat sich für das erfindungsgemäße Nachweisverfahren von Vorteil erwiesen. Bevorzugt ist eine Konzentration von 30 %.

Zur Probenvorbereitung für den Nachweis einer ordnungsgemäß durchgeführten Erhitzung von Milch mit Hilfe der Lactoperoxidase-Aktivität wird die Milch in der Regel mit der Pufferlösung verdünnt. Die entsprechende Probenvorbereitung von Milchprodukten erfolgt indem mit Pufferlösung extrahiert und der Extrakt bei Bedarf anschließend mit der Pufferlösung verdünnt. Üblicherweise sind Verdünnungen zwischen ½ bis 1/20 ausreichend, bei besonders reichhaltigen Proben können sich jedoch auch Verdünnungen bis 1/100 erforderlich erweisen. Dagegen kann bei bestimmten Milchproben, wie z.B. UHT- oder Sterilmilch, unverdünnt gearbeitet werden.

Als Standardlösung wird erfindungsgemäß eine milcheigene Lactoperoxidase-Standard-Lösung verwendet. Üblicherweise wird diese in Form einer Verdünnungsreihe aufgetragen.

Als fester Träger für das erfindungsgemäße Verfahren haben sich insbesondere dünne Schichten wie Membranen, insbesondere modifizierte Cellulosemembranen erwiesen. Besonders geeignet sind hier Cellulose-Nitrat-Membranen.

Nach der Probenvorbereitung werden Proben in der Größenordnung von ca. 1 µl des verdünnten Probenmaterials und des verdünnten Standardmaterials auf den festen Träger aufgetragen und ca. 30 Minuten getrocknet. Die Membran wird anschließend mit wenig Puffer (ca. 20 ml) gewaschen und darauf mit frisch angesetzter Färbelösung, die Substrat in Puffer gelöst enthält sowie einen Zusatz von Wasserstoffperoxid, überschichtet. Die Inkubation der Proben mit der Färbelösung erfolgt bei Raumtemperatur unter Lichtausschluß und ist nach ca. 30-60 Minuten abgeschlossen. Danach wird die Reaktion gestoppt, indem die Membran mit bidestilliertem Wasser gewaschen wird. Die Auswertung erfolgt durch Vergleich der Farbintensitäten der Probenspots mit denen der Spots der Standard-Verdünnungsreihe. Durch das erfindungsgemäße Testsystem ist somit erstmals eine quantitative Auswertung der Enzymaktivität auf festen Trägern möglich.

Die Lactoperoxidase gilt dann als vollständig inaktiv, wenn kein sichtbar gefärbter Spot zu erkennen ist. Ist die Lactoperoxidase inaktiv, so ist der Nachweis für eine ordnungsgemäß durchgeführte Pasteurisierung der Milch bzw. der Milchprodukte erbracht.

Der Aktivitätsnachweis der Alkalischen Phosphatase wird in einem pH-Bereich von ca. 8 - 10,5 durchgeführt, bevorzugt ist ein Bereich von ca. pH 8,5 - 9,0, als ganz besonders geeignet zeigte sich erfindungsgemäß ein pH-Wert von ca. 9,0. Die pH-Werte der Probelösungen und Standardlösungen werden durch in dem vorgenannten pH-Wert-Bereichen eine gute Pufferkapazitäten aufweisende Puffer gewährleistet. Solche Puffer sind dem Fachmann bekannt. Ein möglicher Puffer ist z.B. Tris-Puffer. Die Konzentration der Pufferlösung liegt erfindungsgemäß im Bereich von ca. 0,08 - 0,14 mM, als besonders bevorzugt ist eine Konzentration von 0,1 mM.

Wird als Substrat zum Nachweis der Aktivität der Alkalischen Phosphatase NBT/BCIP (Nitroblautetrazoliniumchlorid/ 5-Brom-4-chlor-3-indolyl-phosphat) so haben sich als besonders geeignet sogenannte NBT/BCIP-Ready-to-use-Tabletten erwiesen. BCIP dient hierbei als Alkalische Phosphatase-Substrat, welches nach seiner Dephosphorylierung oxidativ in einen blauen Indigofarbstoff überführt wird. Als Oxidationsmittel dient NBT, welches bei dieser Reaktion ebenfalls zu einem rötlich-blauen Farbstoff reagiert und damit farbverstärkend wirkt.

Die Probenvorbereitung erfolgt analog zum Aktivitätsnachweis der Lactoperoxidase. Entsprechendes gilt für den zu verwendenden festen Träger.

Als Enzym-Standardlösungen werden milcheigene Alkalische-Phosphatase-Standard-Lösungen verwendet.

Nach der Probenvorbereitung werden Proben in der Größenordnung von ca. 1 µl des verdünnten Probenmaterials und des verdünnten Standardmaterials auf den festen Träger aufgetragen und ca. 30 Minuten getrocknet. Die Membran wird anschließend mit frisch angesetzter Färbelösung, die Substrat in Puffer gelöst sowie gegebenenfalls einen Farbverstärker enthält, überschichtet. Als Substrat wird vorzugsweise BCIP und als Farbverstärker NBT verwendet. Die Inkubation der Proben mit der Färbelösung erfolgt bei Raumtemperatur unter Lichtausschluß und ist nach ca. 30-60 Minuten abgeschlossen. Danach wird die Reaktion gestoppt indem die Membran mit bidestilliertem Wasser gewaschen wird. Die Auswertung erfolgt durch Vergleich der Farbintensitäten der Probenspots mit denen der Spots der Standard-Verdünnungsreihe

Die Alkalische Phosphatase gilt dann als vollständig inaktiv, wenn kein sichtbar gefärbter Spot zu erkennen ist. Ist die Alkalische Phosphatase inaktiv, so ist der Nachweis für eine ordnungsgemäß durchgeführte Dauer- und Kurzzeiterhitzung der Milch bzw. der Milchprodukte erbracht.

Überraschenderweise konnte somit mit Hilfe des erfindungsgemäßen Verfahrens die Aktivität milcheigener Enzyme ohne vorherige Detergenzienzugabe nachgewiesen werden. Dies war nicht zu erwarten, da Milch im Gegensatz zu Blut oder ähnlichen Flüssigkeiten ein Dreiphasensystem mit einem hohen Fettanteil darstellt. Weiterhin konnte auf einen komplizierten Immunoassay bei der erfindungsgemäßen Testführung verzichtet werden. Das native Enzym haftet überraschenderweise trotz der erforderlichen Waschvorgänge auf der Membran und kann dort mit einem präzipitierenden Farbstoff nachgewiesen werden. Überraschend ist weiterhin, daß die Fettbestandteile der Milch, die den Test stören können, mit einem wässrigen Puffer abgewaschen werden können. Somit wird durch die vorliegende Erfindung ein ungewöhnlich robuster und einfacher Test zur Verfügung gestellt, der eine quantitative Auswertung ermöglicht.

Die nachfolgenden Beispiele dienen einer weiterführenden Erläuterung der vorangegangenen Beschreibung.

### Beispiel 1

### Hocherhitzte Milch-Probe

Die Probe wurde mit Natriumphosphat-Puffer, 50mM, pH 6.5, verdünnt (Verdünnungsreihe 1:2/10/100/1000). Mit Lactoperoxidase-Standard und Natriumphosphat-Puffer wurde eine Verdünnungsreihe hergestellt. Anschließend wurde sofort 1 µl pro Verdünnung auf eine Cellulose-Nitrat-Membran aufgetragen und 30 min getrocknet. Die Membran wurde danach mit 20 ml Puffer gewaschen. Mit frisch angesetzter Färbelösung die 4-(1,4,7,10-Tetraoxadecyl)-1-naphtol in Puffer gelöst sowie Wasserstoffperoxid enhält, wurde dann die Membran überschichtet. Die Membran blieb anschließend 30-60 Minuten bei Raumtemperatur unter Lichtausschluß und wurde danach mit bidestilliertem Wasser gewaschen. Die Probenspots wiesen im Gegensatz zu den Spots der Standardlösung keine Färbung auf Das heißt, daß die pasteurisierte Milch keine aktive Lactoperoxidase enthielt und somit die Wärmebehandlung korrekt durchgeführt worden ist.

### Unbehandelte Milch-Probe

Der Test für den Nachweis der Lactoperoxidase-Aktivität der unbehandelten Milch-Probe wurde analog zum oben beschriebenen Test der pasteurisierten Milch-Probe durchgeführt. Die Probenspots der unbehandelten Milchprobe wiesen die gleiche Färbung auf wie die Spots der Standardlösung. Das heißt, daß die unbehandelte Milch aktive Lactoperoxidase enthielt.

### Beispiel 2

### Kurzzeiterhitzte Milch-Probe

Mit dem Alkalischen Phosphatase (AP)-Standard und mit Tris-Puffer, 0.1 M, pH 9.0, wurde eine Verdünnungsreihe hergestellt (Verdünnungsreihe: 1:2/10/100/1000). Die Probe der kurzzeiterhitzen Milch wurde mit dem Tris-Puffer analog wie oben für den Standard beschrieben verdünnt. Die verdünnte Probe sowie die Verdünnungsreihe des (AP)-Standards wurden anschließend aufeine Cellulosenitrat-Membran aufgetragen. Nach dem 20 - 30 minütigen Trocknen der Membran wurde frisch angesetzte Färbelösung dazugegeben. Die Färbelösung wurde aus sogenannten NBT/BCIP-Ready-to-use-Tabletten durch Auflösen in Wasser hergestellt (NBT/BCIP-Ready-to-use-Tabletten sind bei Boehringer Mannheim GmbH erhältlich). Diese Tabletten enthalten 4-Nitroblautetrazoliumchlorid (NBT) und 5-Brom-4-chlor-3-indolylphosphat, Toluidin-Salz (BCIP). Unter Lichtausschluß wurde dann die Membran bei Raumtemperatur inkubiert. Die Probenspots der kurzzeiterhitzten Milch zeigten im Gegensatz zu den Spots der AP-Standard-Verdünnungsreihe keine Verfärbung. Das heißt, daß die kurzzeiterhitzte Milch keine aktive Alkalische Phosphatase enthielt und somit die Wärmebehandlung korrekt durchgeführt worden ist.

### Unbehandelte Milch-Probe

Der Test für den Nachweis der Alkalischen Phosphatase -Aktivität der unbehandelten Milch-Probe wurde analog zum oben beschriebenen Test der UHT Milch-Probe durchgeführt. Die Probenspots der unbehandelten Milchprobe wiesen die gleiche blaue Färbung auf wie die Spots der Standardlösung. Das heißt, daß die unbehandelte Milch aktive Alkalische Phosphatase enthielt.

## Patentansprüche

1. Verfahren zum Nachweis der korrekt durchgeführten Erhitzungsbehandlung von Milch und Milchprodukten mit Hilfe eines enzymatischen Verfahrens auf einem festen Träger, dadurch gekennzeichnet, daß eine entsprechende Probe in einer gepufferten Lösung mit einem oder mehreren milchspezifischen Enzymsubstraten auf einem festen Träger vereinigt wird wobei ein einen präzipitierenden Farbstoffbildendes Enzymsubstrat verwendet wird.

2. Verfahren gemäß Anspruch 1, dadurch gekennzeichnet, daß Lactoperoxidase und / oder alkalischen Phosphatase bestimmt wird.

3. Verfahren gemäß einem der Ansprüche 1 - 2, dadurch gekennzeichnet, daß Lactoperoxidase bestimmt wird.

4. Verfahren gemäß Anspruch 3, dadurch gekennzeichnet, daß als Enzymsubstrat ein Naphtolderivat verwendet wird.

5. Verfahren gemäß Anspruch 4, dadurch gekennzeichnet, daß 4-(1,4,7,10-Tetraoxadecyl)-1-naphtol verwendet wird.

6. Verfahren gemäß einem der Ansprüche 1 - 2, dadurch gekennzeichnet, daß als Substrat für die alkalische Phosphatase ein Phosphatgruppen aufweisendes Indol verwendet wird.

7. Verfahren gemäß Anspruch 6, dadurch gekennzeichnet, daß 5-Brom-4-chlor-3-indolylphosphat verwendet wird.

8. Verfahren gemäß Anspruch 7, dadurch gekennzeichnet, daß zusätzlich Nitroblautetrazoliniumchlorid als Farbverstärker zugegen ist.

9. Verfahren nach einem der Ansprüche 1-8, dadurch gekennzeichnet, daß der Träger aus einer Membran besteht.

10. Verfahren nach Anspruch 9, dadurch gekennzeichnet, daß der Träger aus einer modifizierten Cellulose-Membran besteht.

11. Verfahren gemäß Anspruch 10, dadurch gekennzeichnet, daß eine Cellulosenitrat-Membran als Träger verwendet wird.

12. Verfahren nach einem der Ansprüche 1 - 11, dadurch gekennzeichnet, daß als Vergleichslösung milcheigene Enzym-Standards verwendet werden.

13. Testkit bestehend aus einem Puffer, einem einen präzipitierenden Farbstoffbildenden Naphtolderivat, einer modifizierten Cellulose-Membran sowie milcheigenen Enzym-Standards.

14. Testkit bestehend aus einem Puffer, einem einen präzipitierenden Farbstoffbildenden Phosphatgruppen aufweisenden Indol, einem Farbverstärker, einer modifizierten Cellulose-Membran sowie milcheigenen Enzym-Standards.
